# EUROPEAN PATENT SPECIFICATION

(11) **EP 0 561 087 B1**
(45) Date of publication and mention of the grant of the patent: **04.08.1999**
(21) Application number: 92400771.9
(22) Date of filing: 20.03.1992
(51) Int. Cl.: C07K 14/00, C12N 15/15, C12P 21/00, C12P 21/08, C12Q 1/68, G01N 33/53, C12N 15/00

(54) **Mutated form of the beta-amyloid precursor protein gene**
Mutierte Form von dem Beta-Amyloidprecursor Proteine Gen
Forme mutée du gène de la protéine du précurseur beta-amyloide

(43) Date of publication of application: 22.09.1993
(73) Proprietor: N.V. INNOGENETICS S.A., 9052 Gent (BE)
(72) Inventor: Van Broeckhoven, Christine Neurogenetics Lab., Universiteitsplein 1 B-2610 Antwerpen (BE); Martin, Jean-Jacques Neuropathology Lab., Universiteitsplein 1 B-2610 Antwerpen (BE); Hendriks, Lydia Neurogen.Lab. Born-Bunge Found., Universiteitsplein 1 B-2610 Antwerpen (BE); Cras, Patrick Neurobiology Lab., Universiteisplein 1 B-2610 Antwerpen (BE)
(74) Representative: De Clercq, Ann

(56) References cited:
- WO-A-89/06689
- WO-A-89/07657
- WO-A-90/14840
- SCIENCE. vol. 248, no. 4959, 1990, LANCASTER, PA US pages 1124 - 1126 E.LEVY ET AL. 'Mutation of the Alzheimer's disease...'
- GENE. vol. 87, no. 2, March 1990, AMSTERDAM NL pages 257 - 263 SHUN-ICHI YOSHIKAI ET AL. 'Genomic organization ...'
- BIOCHEMICAL AND BIOPHYSICAL RESEARCH COMMUNICATIONS vol. 178, no. 3, 1991, DULUTH MINNESOTA US pages 1141 - 1146 KATSUJI YOSHIOKA ET AL. 'The 717 val...'

## Description

The present invention relates to a mutated form of the β-amyloid precursor protein gene.

Alzheimer's disease (AD) is a degenerative disorder of the human central nervous system characterized by progressive impairment of memory and intellectual function, usually beginning in middle to late adult life.

AD produces a progressive neuronal degeneration of selective cells in the association and memory areas of the cerebral cortex, combined with similar abnormalities in certain subcortical nuclei. Neuronal loss especially affects the large pyramidal cells of the hippocampus, the amygdala and the parietal and frontal association area. Histopathologically, many of the degenerating nerve cells contain tangles of twisted intracellular fibrils of a unique protein configuration related to altered neurofibrillary protein, as well as amyloid plaques and cerebrovascular amyloid deposits comprising extracellular insoluble fibrillar proteins which occur in abnormally large numbers (Wong, C.W., et al., Proc. Natl. Acad. Sci. USA 82:8729 (1985); Hardy, J.A., et al., Neurobiol. Aging 7:489 (1986)). These findings have become a hallmark of the disease.

Recent studies have begun to elucidate the biochemical basis of the disorder. A 42-amino acid peptide has been isolated as the major constituent of brain amyloid plaques and deposits of patients afflicted with AD (Glenner, G.G. and Wong, C.W., Biochem. Biophys. Res. Commun. 122:885 (1984) and Masters, C.L., et al., Proc. Natl. Acad. Sci. USA 82:4245 (1985)). This peptide has been sequenced, and has a partial beta-pleated sheet structure; hence, it is called a β-amyloid peptide or BAP.

Molecular genetic studies (primarily cDNA cloning, but also immunostaining) indicate that the β-amyloid peptide is derived from a larger amyloid precursor protein, called APP. Using a fetal brain library and an oligonucleotide probe drawn from the amino acid sequence, what appears to be a full-length cDNA clone with a 695-amino acid reading frame for the putative precursor protein has been isolated and called APP 695 (Kang, J., et al., Nature 325:733 (1987)). Other amyloid peptide precursor cDNAs with open reading frames have been subsequently isolated from other SV40-transformed libraries; a 751-amino acid putative precursor (APP 751) both from an SV40-transformed fibroblast library and a promyelocytic leukemia cell line (HL60) library (Ponte, P., et al., Nature 331:525 (1988) and Tanzi, R., et al., Nature 331:528 (1988)), and a 770-amino acid putative precursor (APP 770) from a human glioblastoma library (Kitaguchi, N., et al., Nature 331:530 (1988)). More recently, a fourth protein precursor, APP 563, was described (de Sauvage, F. and Octave, J.N., Science 245:651 (1989); it has the 208 amino acid carboxy terminal containing the BAP domain of APP 751 replaced with a novel 20-amino acid insert. These similar but nonidentical precursors probably arise through differential splicing of transcripts (Tanzi, see above; Ponte, see above; and Lemaire, H.G., et al., Nucleic Acid Res. 17:517 (1989)).

It should be noted that the APP 770 gene is constituted by 18 exons of which exons 16 and 17 encode the sequence for β-amyloid. Mutations in the APP gene have already been described. Said mutations have been reported only in exon 17.

Single base changes have been reported in exon 17 of the β-amyloid precursor protein gene (APP) in familial AD and in hereditary cerebral hemorrhage with amyloidosis Dutch type (HCHWA-D), an autosomal dominant form of cerebral amyloid angiopathy (CAA). In AD, three different amino acid substitutions were found at codon 717, while in HCHWA-D an amino acid at codon 693 was changed (Levy, E., et al., Science 248:1124 (1990); Chartier-Harlin, M.C., et al., Nature 353:844 (1991); Goate, A., et al., Nature 349:704 (1991) and Murrell, J., et al., Science 254:97 (1991)).

The present invention is based upon the finding of a mutation in exon 17 of the APP 770 gene in patients showing progressive presenile dementia and others suffering from a cerebral hemorrhage due to cerebral amyloid angiopathy.

In this family, the mutation may be responsible for the progressive deposition of β-amyloid (βA) (Glenner, G.G. and Wong, C.W., Biochem. Biophys. Res. Commun. 122:885 (1984) and Masters, C.L., et al., Proc. Natl. Acad. Sci. USA 82:4245-4249 (1985)), a 4-kDa proteolysis product of APP (Kang, J., et al., Nature 325:733 (1987)) in the blood vessel walls and in the brain parenchyma.

It is therefore an object of the present invention to provide a protein which is a raw material for producing an antibody which is useful for the diagnosis of abnormal APP processing related to the formation of amyloid plaques.

It is another object of the present invention to provide a nucleotide sequence coding for the above-mentioned protein which is useful for producing the protein by recombinant techniques.

It is still another object of the present invention to provide an antibody specific for the above-mentioned protein which is useful for the diagnosis of abnormal APP processing related to the formation of amyloid plaques.

It is a further object of the present invention to provide a DNA probe or an RNA probe useful for determining the amount of an mRNA coding for a substance causing the abnormal APP processing related to the formation of amyloid plaques in a brain cell or any cells or cell lines suitable for the study of brain metabolism for the diagnosis of the above-mentioned brain abnormality.

It is still a further object of the present invention to provide a DNA probe or an RNA probe useful for detecting a specific base sequence in a chromosomal DNA, which relates to the production of a substance involved in the formation of amyloid plaques.

It is a further object of the present invention to provide a chromosomal DNA fragment coding for mutated β-amyloid which can be used for producing a pathological model animal such as a transgenic animal for use
- in research on the deposition of β-amyloid in the blood vessel walls and in the brain parenchyma and
- in determining the relationship between the deposition of β-amyloid and presenile dementia and between β-amyloid deposition and cerebral hemorrhage due to cerebral amyloid angiopathy,
- and for the isolation of transgenic cells useful for immortalization and for drug study in presenile dementia and cerebral hemorrhage.

The invention relates to a polypeptide containing a sequence of contiguous amino acids of the polypeptide sequence coded by exon 17 of the cDNA of the APP 770 gene, with said sequence of contiguous amino acids being such that:
- it has from 5 to the total number of amino acids coded by said exon 17,
- and it contains the amino acid corresponding to codon 692 in the cDNA of the APP 770 gene and which is alanine substituted for glycine.

APP 770 gene is described in Kitaguchi et al., Nature 311:530 (1988). Exon 17 is described in Yoshikai S., et al., Gene 87:257 (1990).

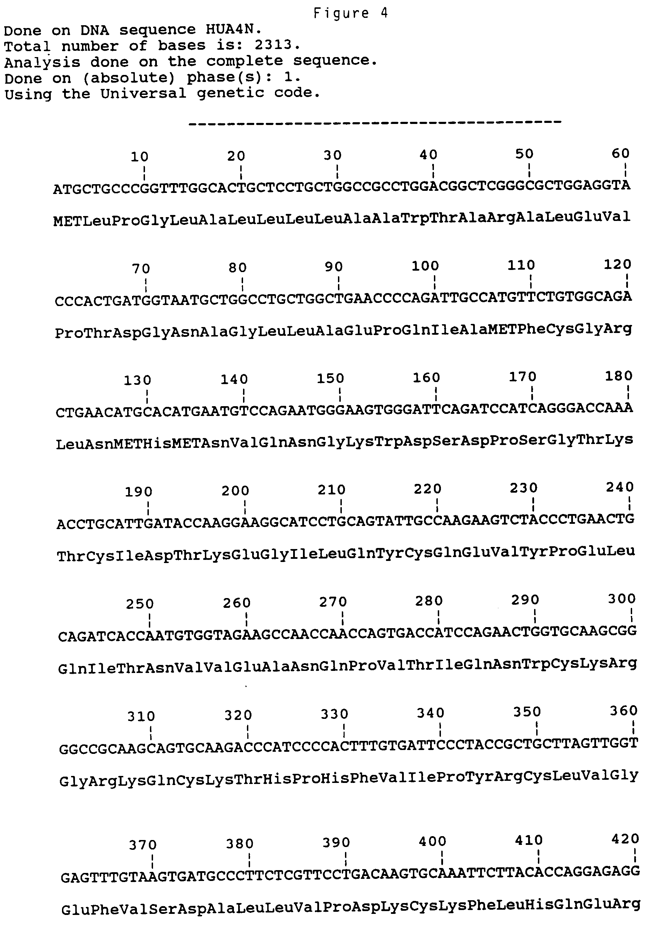

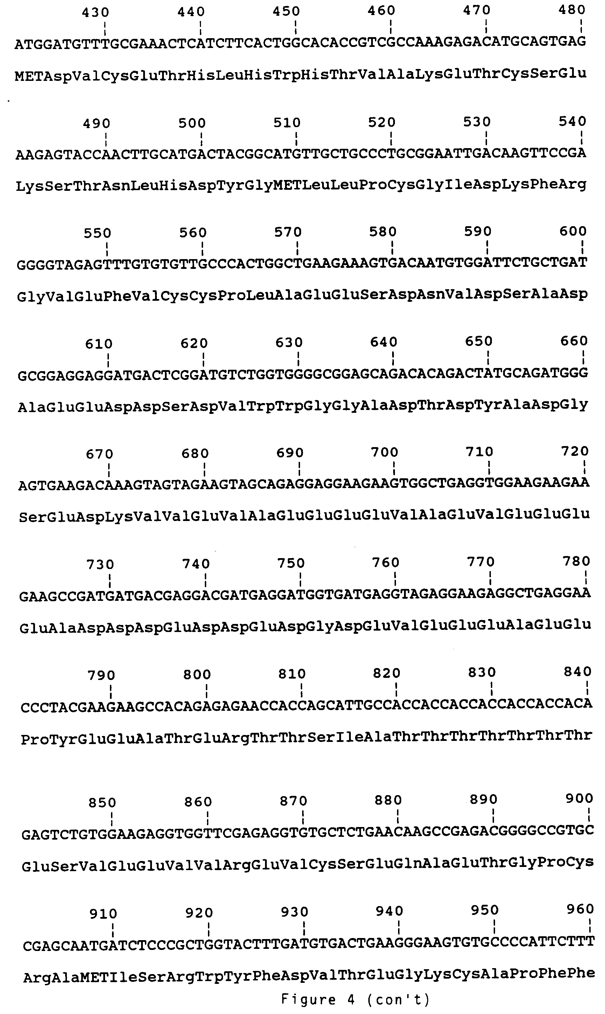

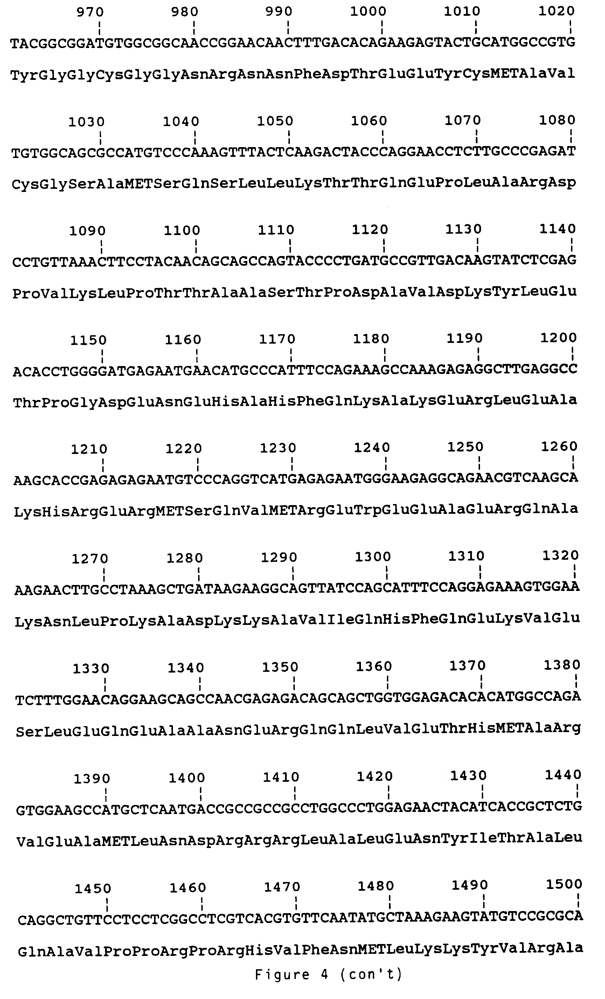

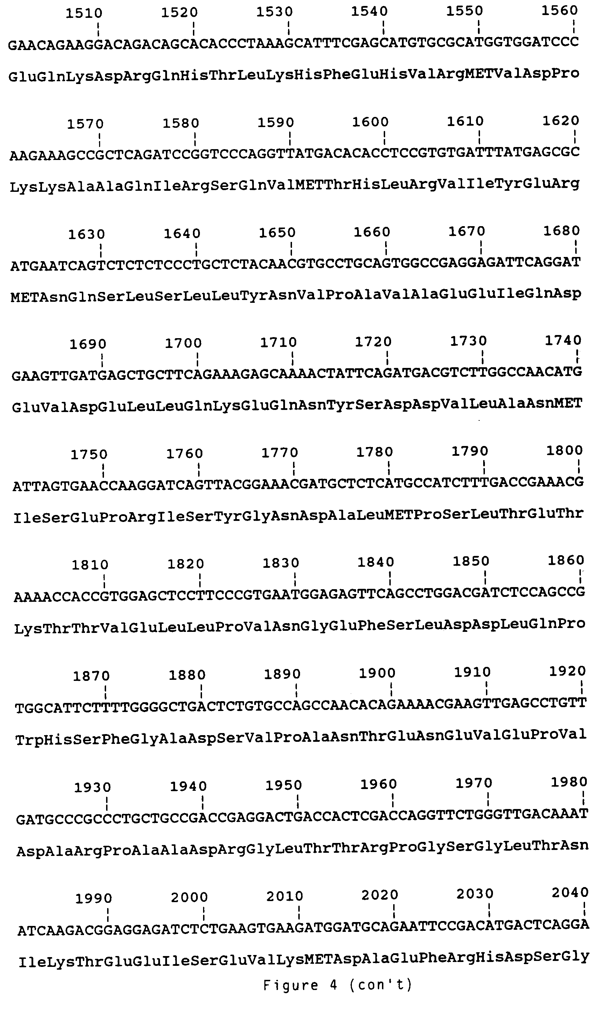

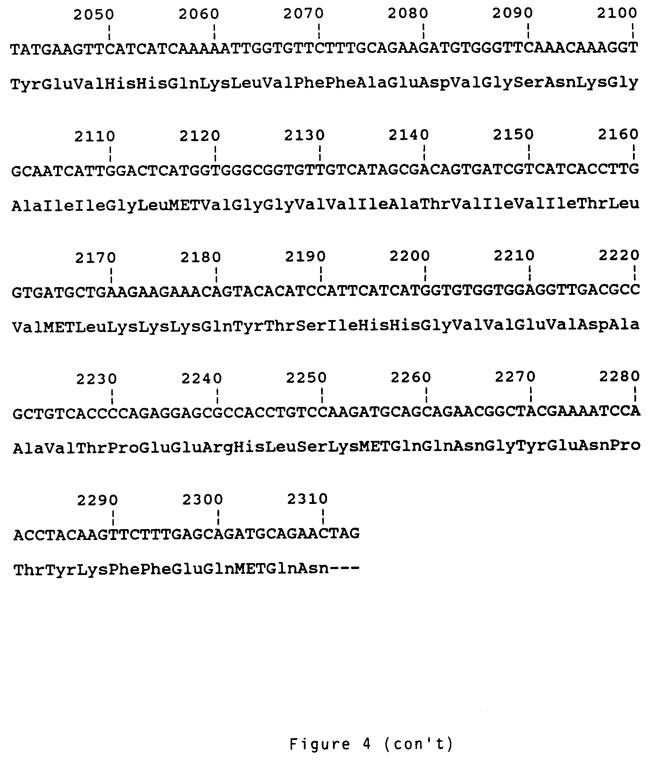

The nucleotide sequence and amino acid sequence of APP 770 are deduced from the reference of Kitaguchi et al. (see Supra) and Kang, J., et al., Nature 325:733 (1986) and are represented in Figure 4.

According to an advantageous embodiment of the invention, the polypeptide contains a sequence of contiguous amino acids of β-amyloid, with said sequence of contiguous amino acids being such that:
- it contains from 5 to the total number of amino acids of β-amyloid,
- and it contains the amino acid corresponding to codon 692 in the cDNA of the APP 770 gene and which is alanine substituted for glycine.

The APP gene is composed of 18 exons, of which exons 16 and 17 encode the sequence for the 42 amino acids of the β-amyloid (Yoshikai, S., et al., Gene 87:257 (1990). The β-amyloid is partly inserted into the transmembrane domain with 28 amino acids protruding into the extracellular space (Kang, J., et al., Nature 325:733 (1987). The mutation described causes a substitution in the extracellular part of β-amyloid at amino acid 21, changing an alanine into a glycine (Ala ---> Gly). The mutation is located next to the HCHWA-D mutation which substitutes a glutamic acid for a glutamine (Glu ---> Gln) at amino acid 22 of β-amyloid (Levy, E., et al., Science 248:1124 (1990).

An advantageous polypeptide of the invention contains a sequence of contiguous amino acids of the APP 770 transcript, with said sequence of contiguous amino acids being such that:
- it has from 5 to the total number of amino acids of APP 770 transcript, and
- it contains the amino acid corresponding to codon 692 in the cDNA of the APP 770 gene, and which is alanine substituted for glycine, and more particularly the following polypeptide: Val-Phe-Phe-Gly-Glu-Asp-Val-Gly, or a polypeptide of 30 amino acids containing the above-mentioned polypeptide.

The invention also relates to a nucleic acid sequence characterized by the fact that it codes for anyone of the polypeptides according to the invention. The invention also relates to a nucleic acid containing a nucleotide sequence of contiguous nucleotides of exon 17, with said nucleotide sequence of contiguous nucleotides being such that:
- it has from 10 to the total number of nucleotides of exon 17 from the cDNA of the APP 770 gene,
- and it contains the nucleotide corresponding to position 2075 in the cDNA of the APP 770 gene, and which is C substituted by G.

The invention also relates to a nucleic acid containing a nucleotide sequence of contiguous nucleotides of exons 16 and 17, with said nucleotide sequence of contiguous nucleotides being such that:
- it has from 10 to the total number of nucleotides of exons 16 and 17 of the cDNA of the APP 770 gene,
- and it contains the nucleotide corresponding to position 2075 in the cDNA of the APP 770 gene, and which is C substituted by G.

The invention also relates to a nucleic acid containing a nucleotide sequence of contiguous nucleotides of the cDNA of the APP 770 gene, with said nucleotide sequence of contiguous nucleotides being such that:
- it has from 10 to the total number of nucleotides of the cDNA of the APP 770 gene,
- and it contains the nucleotide corresponding to position 2075 in the cDNA of the APP 770 gene, and which is C substituted by G,
   and more particularly the following oligonucleotides:
   GTTCTTTGGAGAAGATG
   TTCTTTGGAGAAGAT,
   or nucleic acids containing the above-mentioned oligonucleotides.

The invention also relates to a nucleic acid containing a nucleotide sequence according to the invention inserted into a heterologous nucleic acid.

The invention also relates to a recombinant vector, particularly for cloning and/or expression, comprising a vector sequence, notably of the type plasmid, cosmid, phage DNA or virus DNA, and a recombinant nucleic acid according to the invention, in one of the nonessential sites for its replication.

The invention also relates to a recombinant vector containing in one of its nonessential sites for its replication necessary elements to promote the expression of polypeptides according to the invention in a cellular host and particularly a promoter recognized by the RNA polymerase of the cellular host, particularly an inducible promoter and possibly a sequence coding for transcription termination signals and possibly a signal sequence and/or an anchoring sequence.

The invention also relates to a recombinant vector containing the elements enabling the expression by E. coli of a nucleic acid according to the invention inserted in the vector, and particularly the elements enabling the expression of the gene of the invention or part thereof as a mature protein, or as part of a fusion protein containing the necessary signals for transport to the periplasmic space, with said elements possibly containing amino acid sequences to facilitate downstream processing of the protein coded by said recombinant vector.

The invention also relates to a cellular host which is transformed by a recombinant vector according to the invention, and containing the regulatory elements enabling the expression of the nucleotide sequence coding for the polypeptide of the invention in this host, as a mature protein or as part of a fusion protein, the fusion moiety of which is used in the fusion protein being a part of a non-homologous protein chosen to optimize the expression of the fusion protein, or as part of a fusion protein containing the necessary signals either for transport to the periplasmic space or for secretion in the medium of the cellular host, with said elements possibly containing amino acid sequences to facilitate downstream processing of the protein coded by said recombinant vector.

The invention also relates to a cellular host chosen from among bacteria such as E. coli, transformed by the vector according to the invention or chosen from among eukaryotic organism, transfected by a vector of the invention.

The invention also concerns an expression product of a nucleic acid expressed by a transformed cellular host according to the invention.

The invention also concerns an antibody characterized by the fact that it is directed against a recombinant polypeptide containing an epitope presenting the mutation of the invention and characterized by the fact that it does not recognize non-mutated exon 17 of the APP 770 gene at position 2075 and which does not recognize the non-mutated APP 770 gene at position 2075.

It should be noted that the expression "non-mutated (exon 17 or APP gene) at position 2075" means that position 2075 is occupied by C, which does not exclude the fact that other positions can contain mutations.

The invention also relates to nucleotide probes, hybridizing with anyone of the nucleic acids of the invention or with their complementary sequences, under hybridization conditions such that they do not hybridize with the non-mutated APP gene (at position 2075) or with non-mutated exon 17 (at position 2075) or with messenger RNA of non-mutated APP 770 gene (at position 2075) or of messenger RNA of non-mutated exon 17 (at position 2075), and more particularly the following ones:
GTTCTTTGGAGAAGATG
TTCTTTGGAGAAGAT.

The invention also relates to a process for preparing a recombinant polypeptide according to the invention comprising the following steps:
- the culture in an appropriate medium of a cellular host which has previously been transformed by an appropriate vector containing a nucleic acid of the invention, and
- the recovery of the polypeptide produced by the above-said transformed cellular host from the above-said culture.

The invention also relates to a method for the determination of the mutation at position 2075 (with respect to the cDNA of the APP 770 gene) in the APP 770 gene or part of it, comprising the following steps:
- the possible previous amplification of the amount of the nucleotide sequences of the invention, liable to be contained in a biological sample, taken from said patient, by means of a DNA primer set,
- contacting the above-mentioned biological sample with a nucleotide probe according to the invention, under conditions enabling the production of an hybridization complex formed between said probe and said nucleotide sequence,
- detecting the above-said hybridization complex which has possibly been formed.

The biological sample can be blood lymphocytes, brain tissue, skin, subcutaneous tissue, intestine, cells from neuronal origin, meningocerebral blood vessels, hair roots, and mouth washings or extracellular cerebral deposits (amyloid plaques).

A mutation sequence can be distinguished from the corresponding wild type sequence using a variety of hybridization protocols. By way of example and not intended to be limiting, a typical protocol for the detection of a particular nucleic acid sequence bound to a solid support (e.g. a nitrocellulose membrane) is described below.

The membranes were prehybridized in a mixture containing the following components: 3 x SSC (1 x SSC is 0.15 M NaCl plus 0.015 M sodium citrate, pH 7.0), 25 mM sodium phosphate buffer (pH 7.1), 20% (v/v) deionized formamide, 0.02% Ficoll (type 400, Sigma), 0.02% bovine serum albumin, 0.02% polyvinylpyrrolidone, 0.1 mg sheared heat-denatured salmon sperm DNA ml⁻¹, and 0.2% SDS, usually for 0.5 - 1 h at the appropriate temperature. The hybridization mixture had the same composition except that approximately 10⁶ c.p.m. of ³²P-labelled probe ml⁻¹ was added. Hybridizations were performed at the same temperature for 1-2 h. The membranes were washed for 30 min in 3 x SSC, 25 mM sodium phosphate buffer (pH 7.1), 20% (v/v) deionized formamide, 0.2% SDS at the hybridization temperature.

The optimal hybridization and wash temperature is a function of several parameters such as the nature of the probe (RNA or DNA; length; %G+C; secondary structure, etc.) and the composition of the hybridization and wash solution (ionic strength, presence of hybrid-destabilizing agents, etc.).

For a probe of the invention with the following sequence: GTTCTTTGGAGAAGATG, the appropriate hybridization temperature and wash temperature in the above defined medium is about 43°C.

Another method for the determination of the mutation at position 2075 (with respect to the cDNA of the APP 770 gene) in the APP 770 gene or part of it, comprises the following steps:
- the possible previous amplification of the amount of a nucleotide sequence according to the invention, liable to be contained in a biological sample, taken from a patient, by means of a DNA primer set in the presence of radioactive nucleotides,
- the denaturation of the amplified nucleotide sequence to split it into two strands,
- sequencing one strand of the obtained denaturated nucleotide sequence,
- and possibly checking the sequencing of said strand of nucleotide sequence containing the mutation by sequencing of the opposite strand.

This above-mentioned method, which is the direct sequencing method for the determination of the mutation in affected individuals, is described hereafter in the examples.

Another method for the determination of the mutation at position 2075 (with respect to the cDNA of the APP 770 gene) in the APP 770 gene or part of it comprises the following steps:
- the amplification of the amount of a nucleotide sequence of the invention , liable to be contained in a biological sample taken from a patient, by means of a DNA primer set,
- the denaturation of the amplified nucleotide sequence to split it into two strands,
- and the separation of bands, one corresponding to the strand of the nucleotide sequence containing the mutation and the other one corresponding to the strand of the nucleotide sequence containing no mutation.

It is appropriate to carry out the same analysis on a control sample taken from an unaffected patient.

The above-mentioned method applies when the patient is heterozygous. When the patient is homozygous, it is necessary to compare the band which has been obtained with that obtained from a sample taken from an unaffected patient.

This above-mentioned method, which is the single-strand conformational analysis for the determination of the mutation in affected individuals, is described hereafter, in the examples.

Another method for the determination of the mutation at position 2075 (with respect to the cDNA of the APP 770 gene) in the APP 770 gene or part of it comprises the following steps:
- the amplification of the amount of the nucleotide sequences of the invention, liable to be contained in a biological sample, taken from a patient, by means of a DNA primer set,
- the digestion with the restriction enzyme CviRI liable to cleave the CviRI site which is present in the non-mutated form and absent in the mutated form,
- the separation of the bands, resulting from the digestion, for instance on a polyacrylamide or on an agarose gel,
- the detection of the bands, for instance with ethidium bromide.

This above-mentioned method corresponds to the detection of the mutation after restriction enzyme digestion.

Another method for the determination of the mutation at position 2075 (with respect to the cDNA of the APP 770 gene) in the APP 770 gene or part of it comprises the following steps:
- sequencing of the amino acids of the invention, with said amino acids being liable to be contained in a biological sample taken from a patient, for instance according to the method described in Prelli, F., et al., Biochem. Biophys. Res. Commun. 170:301 (1990).

It is possible to isolate amyloid fibrils from the vascular plaques or from plaques present in brain parenchyma.

A diagnostic assay to detect the presence of the mutation in amyloid fibrils (from the vascular plaques) can be the following.

### - Protein isolation and purification:

Leptomeningeal amyloid fibrils were isolated from patients, as previously described in Van Duinen, S.G., et al. (Proc. Natl. Acad. Sci. USA 84:5991 (1987)) and Prelli, F., et al. (Biochem. Biophys. Res. Commun. 151:1150 (1988)). Briefly, they were solubilized in 6 M guanidine hydrochloride/0.1 M Tris/0.17 M dithiothreitol, pH 10.2, and stirred for 48 h at room temperature. After addition of 25% (v/v) of 2 M guanidine hydrochloride/4 M acetic acid, the solution was applied to a calibrated Sephadex G-100 column, 2.5 x 10 cm equilibrated with 5 M guanidine hydrochloride/1 M acetic acid. The purity and molecular weight of the fractions were determined on 17% polyacrylamide gels containing 0.1% sodium dodecyl sulfate (SDS) (Laemmli U.K., Nature 227:680 (1970)). Samples were pretreated with 88% formic acid for 1 h at room temperature, followed by drying under nitrogen. For Western blot analysis (Towbin, H., et al., Proc. Natl. Acad. Sci. USA 76:4350 (1979)), electrophoretic transfers of gels were carried out as previously described (Prelli, F., et al., Biochemistry 26:8251 (1987)). Polyclonal rabbit antiserum to a synthetic peptide, SP 28 (Castano, E.M., et al., Biochem. Biophys. Res. Commun. 141:782 (1986)), corresponding to the 28 NH₂-terminal residues of AD-vascular β-protein, was used as the first antibody, and peroxidase-conjugated goat anti-rabbit antiserum was visualized by color development with 3,3'-diaminobenzidine and hydrogen peroxide.

### - Enzymatic Digestion:

The amyloid protein was pretreated with 88% formic acid (Masters, C.L., et al., Proc. Natl. Acad. Sci. USA 82:4245 (1985)), dissolved in 0.2 M ammonium bicarbonate, pH 8.2, and incubated (enzyme: substrate, 1:100 wt/wt) for 4 h at 37°C with 1-tosylamido-2-phenylethyl chloromethyl ketone-treated trypsin. Proteolysis was terminated by rapid freezing followed by lyophilization.

### - High-Performance Liquid Chromatography (HPLC):

Tryptic peptides were isolated by reverse-phase chromatography on a µ-Bondapak C₁₈ column (0.78 x 30.0 cm, Waters) with a gradient of 0-66% acetonitrile in 0.1% (v/v) trifluoroacetic acid at pH 2.5. The column eluents were monitored at 214 nm.

### - Amino acid analysis:

Intact and tryptic peptides of the amyloid protein were hydrolyzed for 24 h in vacuo at 110°C with 0.2 ml 6 N HCl containing 0.01% phenol, dried, resuspended in 20 µl of methanol:water:triethylamine solution (2:2:1, v/v) and redried. Pre-column derivatization of the samples were performed by addition of 20 µl of phenylisothiocyanate:water:triethylamine:methanol (1:1:1:7, v/v). After 30 min at room temperature they were dried under vacuum and analyzed in a Waters Pico-Tag amino acid analyzer using the standard Pico-Tag method. The presence of tryptophan was determined by amino acid sequencing.

### - Sequence Analysis:

Sequence analysis was carried out on a 477A microsequencer and the resulting phenylthiohydantoin amino acid derivatives were identified using the online 120A PTH analyzer and the standard program (Applied Biosystems).

A diagnostic assay to detect the presence of the mutation in amyloid fibrils (from brain parenchyma) can be adapted on the protocol described in Masters, C.L. et al., Proc. Natl. Acad. Sci. USA 82:4245 (1985).

Another method for the determination of the mutation at position 2075 (with respect to the cDNA of the APP 770 gene) in the APP 770 gene, or part of it, is the denaturing gradient gel electrophoresis, such as described for instance in Proc. Natl. Acad. Sci. USA, vol. 86, p. 232-236 (January 1989); Genetics and Genomics, vol. 3, p. 217-223 (1988).

Another method for the determination of the mutation at position 2075 (with respect to the cDNA of the APP 770 gene) in the APP 770 gene, or part of it, is temperature gradient gel electrophoresis (TGE) denaturation, such as described for instance in Nucleic Acids Research, vol. 15, number 13, p. 5069-5083 and p. 5085-5103 (1987).

The invention also relates to a method for producing a transgenic non-human mammalian animal having a probability of developing amyloid plaque deposition and/or cerebral hemorrhage, with said method comprising the incorporation of a nucleotide sequence according to the invention into the genome of a non-human mammalian animal.

A transgenic non-human mammalian animal can be prepared according to the protocol described in International Review of Cytology, vol. 115, p. 171-222 (1989).

### DESCRIPTION OF THE FIGURES

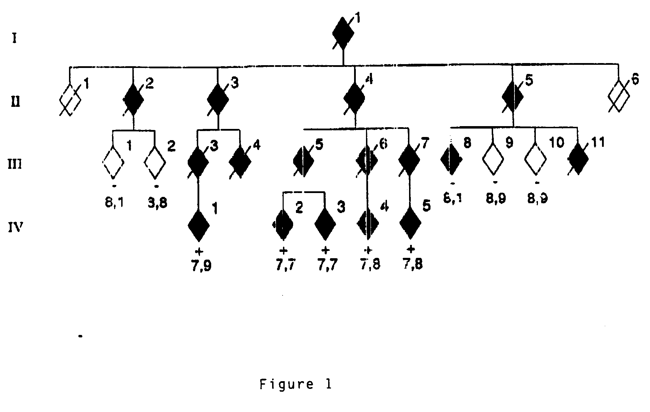

### - Figure 1: Family 1302.

The pedigree is disguised and shows only the escapees and the patients for reasons of confidentiality. The Roman numbers left are the generations, the numbers above the symbols are the individuals. The black symbols represent affected individuals. The diagnosis of the deceased patients was made by heteroanamnesis since autopsy data was not available. The living patients were diagnosed using neurological examination, neuropsychological testing and brain imaging techniques. Individuals III-4, III-11, IV-1 and IV-3 are patients who suffered a cerebral hemorrhage; all other patients are patients with probable Alzheimer's disease. The presence (+) or absence (-) of the mutation as determined by direct sequencing or SSCA as well as the alleles of the 21-GT12 (D21S210) marker are indicated below the symbols.

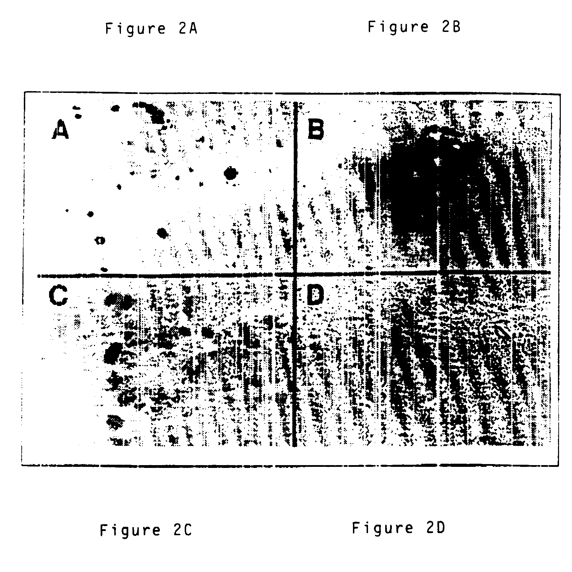

### - Figure 2: Biopsy of patient IV-3.

A, Senile plaques;
B, congophilic angiopathy stained with the polyclonal antiserum β-amyloid 1-42 (Masters, C.L., et al., Proc. Natl. Acad. Sci. USA 82:4245 (1985));
C, Dystrophic senile plaque neurites were labelled with an ubiquitin polyclonal antiserum (Perry, G., et al., Proc. Natl. Acad. Sci. USA 84:3033 (1987));
D, In a semi-serial section, these neurites were negative with AT8, a monoclonal antibody directed against abnormally phosphorylated tau filed at ECACC on October 8, 1991 under No. 91100806.

Methods. Unlabelled antibody bridge or avidin-biotin-labelled immunoperoxidase techniques were used with diaminobenzidine as substrate. The sections were counterstained with hematoxylin.

### - Figure 3:

a, Autoradiography of the sequencing gel from part of the APP 770 exon 17 in individual IV-3 (Fig. 1) and in an unaffected family member showing a single base pair change at position 2075. The C to G transversion leads to an amino acid substitution of an Ala into a Gly at codon 692 of the APP 770 transcript (Yoshikai et al., see supra).

Methods. The conditions and the primers for the PCR amplification of exon 17 were as described (Bakker, E., et al., Am. J. Hum. Genet. 49:518 (1991)). In the amplification, a biotinylated 3' primer was used and the PCR product was bound to streptavidin-coated magnetic beads (Dynal). After DNA denaturation the single-stranded DNA was sequenced (Uhlen, M., Nature 340:733 (1989)) using the Sequenase kit version 2.0 (USB) and the 5' primer. The presence of the mutation was confirmed after sequencing of the opposite strand (data not shown).

b, Single-strand conformational analysis (SSCA) of the mutation at codon 692 in one normal and 6 affected individuals from family 1302 showing the presence of the mutation in all affected, with the exception of one, individual III-8. The numbers refer to the generation number of the individual in the pedigree (Fig. 1).

Methods. The amplification of exon 17 was performed (Bakker et al., see supra) in the presence of [α-³²P)dCTP. After denaturation, the PCR product was loaded on a 5% non-denaturing polyacrylamide gel containing 10% glycerol (Orita, M., et al., Genomics 5:874 (1989)).

### EXAMPLE:

In an epidemiological study of genetic risk factors in Alzheimer's disease (Hofman, A., et al., Neurology 39:1589 (1989)), a four-generation family, coded 1302, was identified with autosomal dominant presenile dementia fulfilling the NINCDS criteria (McKhann, G., et al., Neurology 34:939 (1984)) for probable AD (Fig. 1). The mean age at onset of dementia was 49.3 ± 6.7 years (n = 7, range 41 to 61 years) and the mean age at death 57.7 ± 6.5 years (n = 9, range 47 to 68 years). In the family, were also identified 4 patients with a cerebral hemorrhage at a mean age of 39.5 ± 3.1 years (range 35 to 42 years). Prior to the cerebral hemorrhage these patients did not show signs of dementia. Family 1302 was not related to the known HCHWA-D families.

One patient (IV-3 in Fig. 1) who suffered from a large hemorrhage in the left parieto-occipital cortex was studied in more detail. The hematoma was evacuated and biopsies were taken. Immunohistochemistry on the biopsy material with a β-amyloid polyclonal antiserum, showed extensive amyloid deposition in the blood vessel walls and in parenchymal senile plaques (Fig. 2A and 2B). The senile plaques were predominantly diffuse amyloid deposits but neuritic plaques with a dense amyloid core were also observed. Dystrophic neurites in the senile plaques and in the perivascular amyloid depositions were immunostained by a polyclonal antiserum to ubiquitin (Fig. 2C). However, no neurofibrillary tangles were detected using a monoclonal antibody to pathological tau (Fig. 2D).

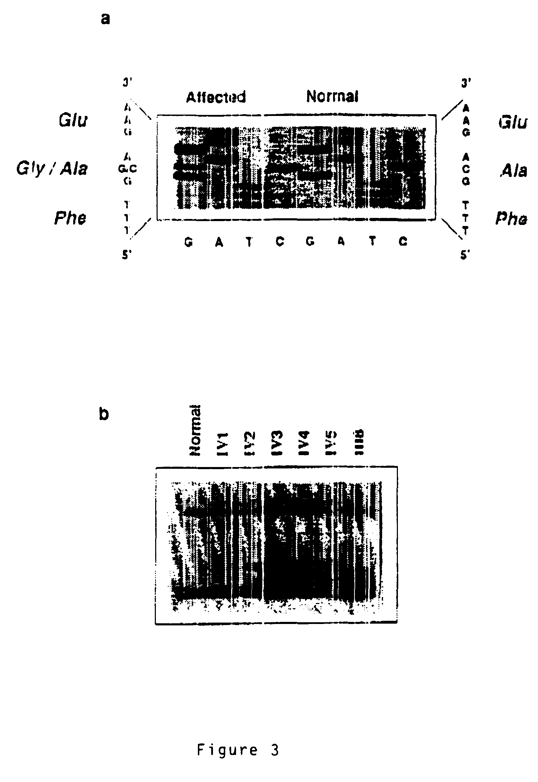

The possible presence of a mutation in exon 17 of the APP gene in patient IV-3 was examined. Leukocyte DNA was used for PCR amplification and direct sequencing (Fig. 3a). Neither the HCHWA-D mutation nor the known AD mutations were found. Instead, a novel C-to-G transversion at position 2075 substituting alanine (Ala) into glycine (Gly) at codon 692 of the APP 770 transcript (Yoshikai, S., et al., Gene 87:257 (1990)) was detected. Direct sequencing and single-strand conformational analysis (SSCA) were used to test for the mutation in the affected individuals. Theoretically the mutation can also be detected after restriction enzyme digestion since the C-to-G transversion destroys a CviRI recognition site. The mutation was present in patient IV-1 with cerebral hemorrhage and in patients IV-2, IV-4, and IV-5, but not in patient III-8, with dementia (Fig. 3b). In the latter patient, the onset at 61 years occurred considerably later than the mean age of onset (47.3 ± 4.6 years, n = 6, range 41 to 55 years) found in the other relatives with dementia, and the current age of 71 years is outside the range of the age at death. Therefore, it is thought likely that the dementia in this patient has a different etiology.

The presence of the mutation in the unaffected individuals by SSCA was also tested. In generation III (Fig. 1), the mutation was absent in III-1, III-2, III-9, and III-10, all individuals who, on the basis of their ages ranging from 64 to 76 years, were judged to have escaped the disease. In generation IV, the age of the 7 unaffected individuals ranged from 38 to 59 years. The mutation was present in 2 of them (individuals not shown for reasons of confidentiality). Possible applications of the mutation in presymptomatic testing are under study, also since in the 2 unaffected individuals carrying the mutation, and currently aged 41 years, suggestive symptoms are starting to occur.

Segregation analysis with the highly polymorphic dinucleotide repeat marker 21-GT12 (D21S210) located close to the APP 770 gene, showed that the mutation segregated with allele 7 (Fig. 1). Allele 7 is not present in individual III-8 indicating that in this patient the dementia recombines with the 21-GT12 marker. The recombination was also observed with the BglII polymorphism detected by the β-amyloid fragment of the APP 770 cDNA (Van Broeckhoven, C., et al., Nature 329:153 (1987)) (data not shown). Linkage was calculated and included all individuals analyzed, except individual III-8. The peak lod score of 2.32 for the 21-GT12 marker and of 3.45 for the mutation at a recombination fraction, θ, of zero (Table 1 below), confirmed that the 21-GT12 marker and the mutation cosegregate with the disease. Further, SSCA showed that the mutation did not occur in 100 normal, unrelated Caucasians.

Table 1 hereafter shows the lod scores for linkage.

**Table 1**

| Recombination fraction θ | | | | | | | |
|---|---|---|---|---|---|---|---|
| | 0.0 | 0.01 | 0.05 | 0.10 | 0.20 | 0.30 | 0.40 |
| APP692 | 3.45 | 3.38 | 3.09 | 2.73 | 2.02 | 1.30 | 0.57 |
| 21-GT12 | 2.32 | 2.26 | 2.00 | 1.68 | 1.11 | 0.58 | 0.14 |

The lod scores were calculated using the MLINK from the LINKAGE package (Lathrop, G., et al., Proc. Natl. Acad. Sci. USA 81:3443 (1984)) assuming a disease frequency of 0.001 and a phenocopy rate of 0.001. The penetrance for the unaffected individuals was estimated from an age at onset curve (Ott, J. in Analysis of Human Genetic Linkage; The Johns Hopkins University Press (eds. Boyer, S.H. et al.) 141-144 (1985)), using a mean age at onset of 44.8 ± 3.3 years calculated in the 5 living patients in generation IV (Fig. 1). The frequency for the APP 692 mutation was 0.01. The allele frequencies of 21-GT12 (D21S210) are available from the Genome Data Base (GDB).

The results obtained suggest that in family 1302 both probable Alzheimer's disease and cerebral hemorrhage are linked to the same mutation at codon 692 of the APP 770 gene. It remains, however, possible that other sequence differences exist in the APP 770 gene since other exons or the promotor sequence in the patients have not yet been analyzed. Nonetheless, mutations other than in exon 17 have not been reported yet.

In the biopsy of patient IV-3, the distribution and quantity of β-amyloid deposits is similar to that seen in HCHWA-D patients (Timmers, W.F., et al., Neurosci. Lett. 118:223 (1990)). Dystrophic neurites were present but no neurofibrillary tangles were observed. However, since autopsy data are not available, the possibility that neurofibrillary tangles will develop as the amyloid deposition progresses cannot be excluded. Normally β-amyloid deposition is prevented since the APP is cleaved within the β-amyloid fragment at amino acid 16 by the APP secretase (Sisodia, S.S., et al. Science 248:492 (1990) and Esch, F.S., et al., Science 248:1122 (1990)). The mutation in family 1302 and in HCHWA-D may alter the efficiency of the APP secretase allowing the processing of APP through alternative pathways that leave the β-amyloid fragment intact. In HCHWA-D families the common clinical manifestation is a cerebral hemorrhage due to extensive cerebral amyloid angiopathy (Haan, J., et al., Arch. Neurol. 47:965 (1990)). In most HCHWA-D patients surviving one or more hemorrhages, dementia was recorded, and some patients showed progressive dementia in the absence of apparent cerebral hemorrhage (Haan, J., et al., Arch. Neurol. 47:649 (1990)). In family 1302, most patients demonstrate dementia in the absence of cerebral hemorrhage.

## Claims

1. Polypeptide containing a sequence of contiguous amino acids of the polypeptide sequence coded by exon 17 of the cDNA of the APP 770 gene, with said sequence of contiguous amino acids being such that:
- it has from 5 to the total number of amino acids coded by said exon 17,
- and it contains the amino acid corresponding to codon 692 in the cDNA of the APP 770 gene and which is alanine substituted for glycine.

2. Polypeptide containing a sequence of contiguous amino acids of β-amyloid, with said sequence of contiguous amino acids being such that:
- it contains from 5 to the total number of amino acids of β-amyloid,
- and it contains the amino acid corresponding to codon 692 in the cDNA of the APP 770 gene and which is alanine substituted for glycine.

3. Polypeptide containing a sequence of contiguous amino acids of the APP 770 transcript, with said sequence of contiguous amino acids being such that:
- it has from 5 to the total number of amino acids of APP 770 transcript, and
- it contains the amino acid corresponding to codon 692 in the cDNA of the APP 770 gene, and which is alanine substituted for glycine,
and more particularly the following polypeptide:
Val-Phe-Phe-Gly-Glu-Asp-Val-Gly,
or a polypeptide of 30 amino acids containing the above-mentioned polypeptide.

4. Nucleic acid sequence characterized by the fact that it codes for anyone of the polypeptides according to claim 1 to 3.

5. Nucleic acid containing a nucleotide sequence of contiguous nucleotides of exon 17, with said nucleotide sequence of contiguous nucleotides being such that:
- it has from 10 to the total number of nucleotides of exon 17 of the cDNA of the APP 770 gene,
- and it contains the nucleotide corresponding to position 2075 in the cDNA of the APP 770 gene, and which is C substituted by G.

6. Nucleic acid containing a nucleotide sequence of contiguous nucleotides of exons 16 and 17, with said nucleotide sequence of contiguous nucleotides being such that:
- it has from 10 to the total number of nucleotides of exons 16 and 17 of the cDNA of the APP 770 gene,
- and it contains the nucleotide corresponding to position 2075 in the cDNA of the APP 770 gene, and which is C substituted by G.

7. Nucleic acid containing a nucleotide sequence of contiguous nucleotides of the cDNA of the APP 770 gene, with said nucleotide sequence of contiguous nucleotides being such that:
- it has from 10 to the total number of nucleotides of the cDNA of the APP 770 gene,
- and it contains the nucleotide corresponding to position 2075 in the cDNA of the APP 770 gene, and which is C substituted by G,
and more particularly the following oligonucleotides:
GTTCTTTGGAGAAGATG
TTCTTTGGAGAAGAT,
or nucleic acids containing the above-mentioned oligonucleotides.

8. Nucleic acid containing a nucleotide sequence according to anyone of claims 4 to 7 inserted into a heterologous nucleic acid.

9. Recombinant vector, particularly for cloning and/or expression, comprising a vector sequence, notably of the type plasmid, cosmid, phage DNA or virus DNA, and a recombinant nucleic acid according to anyone of claims 4 to 7, in one of the nonessential sites for its replication.

10. Recombinant vector according to claim 9, containing in one of its nonessential sites for its replication necessary elements to promote the expression of polypeptides according to anyone of claims 1 to 3 in a cellular host and particularly a promoter recognized by the RNA polymerase of the cellular host, particularly an inducible promoter and possibly a sequence coding for transcription termination signals and possibly a signal sequence and/or an anchoring sequence.

11. Recombinant vector according to claim 10, containing the elements enabling the expression by E. coli of a nucleic acid according to anyone of claims 4 to 7 inserted in the vector, and particularly the elements enabling the expression of the nucleic acid according to anyone of claims 1 to 4, or part thereof as a mature protein, or as part of a fusion protein containing the necessary signals for transport to the periplasmic space, with said elements possibly containing amino acid sequences to facilitate downstream processing of the protein coded by said recombinant vector.

12. Cellular host which is transformed by a recombinant vector according to anyone of claims 10 or 11, and containing the regulation elements enabling the expression of a nucleotide sequence coding for the polypeptide according to anyone of claims 1 to 3 in this host, as a mature protein or as part of a fusion protein, the fusion moiety which is used in the fusion protein being a part of a non-homologous protein chosen to optimize the expression of the fusion protein.

13. Expression product of a nucleic acid according to any of claims 4 to 7 expressed by a transformed cellular host according to any one of claims 12 or 13.

14. Nucleotidic probes, hybridizing with a nucleic acid according to any of claims 4 to 7 or with their complementary sequences, under hybridization conditions such that they do not hybridize with the non-mutated APP gene (at nucleotide position 2075) or with non-mutated exon 17 (at nucleotide position 2075) or with messenger RNA of non-mutated APP 770 gene (at nucleotide position 2075) or of messenger RNA of non-mutated exon 17 (at nucleotide position 2075), and more particularly the following ones: GTTCTTTGGAGAAGATG TTCTTTGGAGAAGAT.

15. Method for the determination of the presence or absence of the mutation at nucleotide position 2075 (with respect to the cDNA of the APP 770 gene) in the APP 770 gene or part of it, comprising the following steps:
- taking of a biological sample from a patient liable to contain the nucleotide sequences according to any of claims 4 to 7,
- possible amplification of the nucleotide sequences according to any one of claims 4 to 7, by means of a DNA primer set,
- contacting the above-mentioned biological sample or the amplified nucleotide sequences contained therein with a nucleotide probe according to claim 16, under conditions enabling the production of an hybridization complex formed between said probe and said nucleotide sequence,
- detecting the above-said hybridization complex which has possibly been formed.
- concluding on the presence or absence of the above mentioned mutation.

16. Method for the determination of the presence or absence of the mutation at nucleotide position 2075 (with respect to the cDNA of the APP 770 gene) in the APP 770 gene or part of it, comprising the following steps:
- taking of a biological sample from a patient liable to contain the nucleotide sequences according to any of claims 4 to 7,
- amplification of a nucleotide sequence according to any one of claims 4 to 7 by means of a DNA primer set in the presence of radioactive nucleotides,
- the denaturation of the amplified nucleotide sequence to split it into two strands,
- sequencing one strand of the obtained denaturated nucleotide sequence,
- and possibly determining the sequence of said strand of the nucleotide sequence containing the mutation by sequencing of the opposite strand.
- concluding on the presence or absence of the above mentioned mutation.

17. Method for the determination of the presence or absence of the mutation at nucleotide position 2075 (with respect to the cDNA of the APP 770 gene) in the APP 770 gene or part of it comprising the following steps:
- taking of a biological sample from a patient liable to contain the nucleotide sequences according to any of claims 4 to 7,
- the amplification of a nucleotide sequence according to any one of claims 4 to 7 by means of a DNA primer set,
- the denaturation of the amplified nucleotide sequence to split it into two strands,
- and the separation of bands, one corresponding to the strand of the nucleotide sequence containing the mutation and the other one corresponding to the strand of the nucleotide sequence containing no mutation.
- concluding on the presence or absence of the above mentioned mutation.

18. Method for the determination of the presence or absence of the mutation. at nucleotide position 2075 (with respect to the cDNA of the APP 770 gene) in the APP 770 gene or part of it comprising the following steps:
- taking of a biological sample from a patient liable to contain the nucleotide sequences according to any of claims 4 to 7 ,
- the amplification of the nucleotide sequences according to any one of claims 4 to 7, by means of a DNA primer set,
- the digestion with the restriction enzyme CviRI liable to cleave the CviRI site, which is present in the non-mutated form and absent in the mutated form,
- the separation of the bands, resulting from the digestion, for instance on a polyacrylamide or on an agarose gel, and
- the detection of the bands, for instance with ethidium bromide.
- concluding on the presence or absence of the above mentioned mutation.

19. Method for the determination of the presence or absence of the mutation at nucleotide position 2075 (with respect to the cDNA of the APP 770 gene) in the APP 770 gene or part of it comprising the following steps:
- taking a biological sample from a patient liable to contain polypeptides according to any of claims 1 to 3
- sequencing of the amino acid of any one of claims 1 to 3,
- concluding on the presence or absence of the above mentioned mutation.

20. Method for producing a transgenic non-human mammalian animal having a probability of developing amyloid plaque deposition and/or cerebral hemorrhage, with said method comprising chromosomally incorporating a nucleotide sequence according to any one of claims 4 to 7 into the genome of a non-human mammalian animal.

## Patentansprüche

1. Polypeptid mit einer Sequenz von aufeinanderfolgenden Aminosäuren der von Exon 17 der cDNA des APP 770-Gens codierten Popypeptidsequenz, wobei diese Sequenz von aufeinanderfolgenden Aminosäuren:
- über 5 bis zu der Gesamtzahl der von Exon 17 codierten Aminosäuren verfügt und
- die dem Codon 692 in der cDNA des APP 770-Gens entsprechende Aminosäure enthält, wobei Glycin durch Alanin substituiert ist.

2. Polypeptid mit einer Sequenz von aufeinanderfolgenden Aminosäuren von β-Amyloid, wobei diese Sequenz von aufeinanderfolgenden Aminosäuren:
- über 5 bis zu der Gesamtzahl der Aminosäuren von β-Amyloid verfügt und
- die dem Codon 692 der cDNA des APP 770-Gens entsprechende Aminosäure enthält, wobei Glycin durch Alanin substituiert ist.

3. Polypeptid mit einer Sequenz von aufeinanderfolgenden Aminosäuren des APP 770-Transkripts, wobei diese Sequenz von aufeinanderfolgenden Aminosäuren:
- über 5 bis zu der Gesamtzahl der Aminosäuren des APP 770-Transkripts verfügt und
- die dem Codon 692 der cDNA des APP 770-Gens entsprechende Aminosäure enthält, wobei Glycin durch Alanin substituiert ist,
ganz besonders das folgende Polypeptid:
Val-Phe-Phe-Gly-Glu-Asp-Val-Gly,
oder ein Polypeptid mit 30 Aminosäuren, das das obengenannte Polypeptid enthält.

4. Nukleinsäuresequenz, dadurch gekennzeichnet, daß sie für ein beliebiges der Polypeptide nach Anspruch 1 bis 3 codiert.

5. Nukleinsäure mit einer Nukleotidsequenz von aufeinanderfolgenden Nukleotiden von Exon 17, wobei diese Nukleotidsequenz von aufeinanderfolgenden Nukleotiden:
- über 10 bis zur Gesamtzahl der Nukleotide von Exon 17 der cDNA des APP 770-Gens verfügt und
- das der Position 2075 in der cDNA des APP 770-Gens entsprechende Nukleotid enthält, wobei C durch G substituiert ist.

6. Nukleinsäure mit einer Nukleotidsequenz von aufeinanderfolgenden Nukleotiden von Exon 16 und Exon 17, wobei diese Nukleotidsequenz von aufeinanderfolgenden Nukleotiden:
- über 10 bis zur Gesamtzahl der Nukleotide von Exon 16 und Exon 17 der cDNA des APP 770-Gens verfügt und
- das der Position 2075 in der cDNA des APP 770-Gens entsprechende Nukleotid enthält, wobei C durch G substituiert ist.

7. Nukleinsäure mit einer Nukleotidsequenz von aufeinanderfolgenden Nukleotiden der cDNA des APP 770-Gens, wobei diese Nukleotidsequenz von aufeinanderfolgenden Nukleotiden:
- über 10 bis zur Gesamtzahl an Nukleotiden der cDNA des APP 770-Gens verfügt und
- das der Position 2075 in der cDNA des APP 770-Gens entsprechende Nukleotid enthält und wobei C durch G ersetzt ist,
ganz besonders die folgenden Oligonukleotide:
GTTCTTTGGAGAAGATG
TTCTTTGGAGAAGAT,
oder Nukleinsäuren, die die obengenannten Oligonukleotide enthalten.

8. Nukleinsäure mit einer Nukleotidsequenz nach einem der Ansprüche 4 bis 7, die in eine heterologe Nukleinsäure insertiert ist.

9. Rekombinanter Vektor, insbesondere für Klonierungs- und/oder Expressionszwecke, mit einer Vektorsequenz, vor allem des Plasmid-, Cosmid-, Phagen-DNA- oder Virus-DNA-Typs, sowie rekombinante Nukleinsäure nach einem der Ansprüche 4 bis 7 in einer der für seine Replikation nicht essentiellen Stellen.

10. Rekombinanter Vektor nach Anspruch 9, der in einer der für seine Replikation nicht essentiellen Stellen erforderliche Elemente zur Förderung der Expression von Polypeptiden nach einem der Ansprüche 1 bis 3 in einer Wirtszelle enthält, insbesondere ein von der RNA-Polymerase der Wirtszelle erkannter Promoter, insbesondere ein induzierbarer Promoter und gegebenenfalls eine für Transkriptionsterminationssignale codierende Sequenz und gegebenenfalls eine Signalsequenz und/oder Ankersequenz.

11. Rekombinanter Vektor nach Anspruch 10 mit den Elementen, die die Expression einer in den Vektor insertierten Nukleinsäure nach einem der Ansprüche 4 bis 7 durch E. coli gestatten, insbesondere die Elemente, die die Expression der Nukleinsäure nach einem der Ansprüche 1 bis 4 oder eines Teils davon als reifes Protein oder als Teil eines die erforderlichen Signale für den Transport in den periplasmatischen Raum enthaltenden Fusionsproteins gestatten, wobei diese Elemente gegebenenfalls Aminosäuresequenzen enthalten, die das Downstreamprocessing des von dem rekombinanten Vektor codierten Proteins erleichtern.

12. Wirtszelle, die mit einem rekombinanten Vektor nach einem der Ansprüche 10 oder 11 transformiert ist und die Regulationselemente, die die Expression einer für das Polypeptid nach einem der Ansprüche 1 bis 3 codierenden Nukleotidsequenz in diesem Wirt als reifes Protein oder als Teil eines Fusionsproteins gestatten, wobei der in dem Fusionsprotein verwendete fusionierte Molekülteil ein Teil eines für die Expressionsoptimierung des Fusionsproteins gewählten nichthomologen Proteins ist.

13. Expressionsprodukt einer Nukleinsäure nach einem der Ansprüche 4 bis 7, das von einer transformierten Wirtszelle nach einem der Ansprüche 12 oder 13 exprimiert wird.

14. Nukleotidsonden, die mit einer Nukleinsäure nach einem der Ansprüche 4 bis 7 oder mit ihren komplementären Sequenzen unter Hybridisationsbedingungen so hybridisieren, daß sie nicht mit dem nichtmutierten APP-Gen (in der Nukleotidposition 2075) oder mit dem nichtmutierten Exon 17 (in der Nukleotidposition 2075) oder mit Boten-RNA des nichtmutierten APP 770-Gens (in der Nukleotidposition 2075) oder mit Boten-RNA des nichtmutierten Exons 17 (in der Nukleotidposition 2075), und ganz besonders den folgenden:
GTTCTTTGGAGAAGATG
TTCTTTGGAGAAGAT
hybridisieren.

15. Verfahren zur Bestimmung des Vorliegens bzw. der Abwesenheit der Mutation an der Nukleotidposition 2075 (in bezug auf die cDNA des APP 770-Gens) in dem APP 770-Gen oder einem Teil davon, das die folgenden Schritte umfaßt:
- Entnahme einer biologischen Probe aus einem Patienten, der gegebenenfalls die Nukleotidsequenzen nach einem der Ansprüche 4 bis 7 enthält,
- gegebenenfalls Amplifikation der Nukleotidsequenzen nach einem der Ansprüche 4 bis 7 unter Verwendung eines DNA-Primer-Sets,
- In-Kontakt-bringen der genannten biologischen Probe oder der darin enthaltenen amplifizierten Nukleotidsequenzen mit einer Nukleotidsonde nach Anspruch 16 unter Bedingungen, die die Bildung eines zwischen der Sonde und der Nukleotidsequenz entstandenen Hybridisationskomplexes gestattet,
- Nachweisen des gegebenenfalls gebildeten genannten Hybridisationskomplexes,
- und Folgern, ob die genannte Mutation vorliegt oder nicht.

16. Verfahren zur Bestimmung des Vorliegens bzw. der Abwesenheit der Mutation in der Nukleotidposition 2075 (in bezug auf die cDNA des APP 770-Gens) in dem APP 770-Gen oder einem Teil davon, umfassend die folgenden Schritte:
- Entnahme einer biologischen Probe aus einem Patienten, der gegebenenfalls die Nukleotidsequenzen nach einem der Ansprüche 4 bis 7 enthält,
- Amplifikation einer Nukleotidsequenz nach einem der Ansprüche 4 bis 7 mittels eines DNA-Primer-Sets in Gegenwart von radioaktiven Nukleotiden,
- Denaturierung der amplifizierten Nukleotidsequenz, wodurch diese in zwei Stränge gespalten wird,
- Sequenzieren eines Strangs der erhaltenen denaturierten Nukleotidsequenz,
- und gegebenenfalls Bestimmung der Sequenz dieses Strangs der die Mutation enthaltenden Nukleotidsequenz durch Sequenzieren des Gegenstrangs,
- und Folgern, ob die genannte Mutation vorliegt oder nicht.

17. Verfahren zur Bestimmung des Vorliegens bzw. der Abwesenheit der Mutation in der Nukleotidposition 2075 (in bezug auf die cDNA des APP 770-Gens) in dem APP 770-Gen oder einem Teil davon, umfassend die folgenden Schritte:
- Entnahme einer biologischen Probe aus einem Patienten, der gegebenenfalls die Nukleotidsequenzen nach einem der Ansprüche 4 bis 7 enthält,
- Amplifikation der Nukleotidsequenzen nach einem der Ansprüche 4 bis 7 mittels eines DNA-Primer-Sets;
- Denaturierung der amplifizierten Nukleotidsequenz, wodurch diese in zwei Stränge gespalten wird; sowie
- Trennung der Banden, wobei eine dem die Mutation enthaltenden Nukleotidsequenzstrang entspricht und die andere dem Nukleotidsequenzstrang, der keine Mutation enthält, entspricht.

18. Verfahren zur Bestimmung des Vorliegens bzw. der Abwesenheit der Mutation in der Nukleotidposition 2075 (in bezug auf die cDNA des APP 770-Gens) in dem APP 770-Gen oder einem Teil davon, umfassend die folgenden Schritte:
- Entnahme einer biologischen Probe aus einem Patienten, der gegebenenfalls die Nukleotidsequenzen nach einem der Ansprüche 4 bis 7 enthält,
- Amplifikation der Nukleotidsequenzen nach einem der Ansprüche 4 bis 7 mittels eines DNA-Primer-Sets,
- Verdauung mit dem Restriktionsenzym CviRI, das die in der nichtmutierten Form vorliegende und in in der mutierten Form fehlende CviRI-Stelle gegebenenfalls schneidet,
- Trennung der aufgrund der Verdauung erhaltenen Banden, zum Beispiel auf einem Polyacrylamid- oder Agarosegel, und
- Nachweis der Banden, zum Beispiel mit Ethidiumbromid, und
- und Folgern, ob die genannte Mutation vorliegt oder nicht.

19. Verfahren zur Bestimmung des Vorliegens bzw. der Abwesenheit der Mutation in der Nukleotidposition 2075 (in bezug auf die cDNA des APP 770-Gens) in dem APP 770-Gen oder einem Teil davon, umfassend folgende Schritte:
- Entnahme einer biologischen Probe von einem Patienten, der gegebenenfalls Polypeptide nach einem der Ansprüche 1 bis 3 enthält,
- Sequenzieren der Aminosäure nach einem der Ansprüche 1 bis 3,
- und Folgern, ob die genannte Mutation vorliegt oder nicht.

20. Verfahren zur Herstellung eines transgenen nichtmenschlichen Säugetiers, das wahrscheinlich eine Amyloid-Plaque-Ablagerung und/oder eine Hirnblutung entwickelt, bei dem eine Nukleotidsequenz nach einem der Ansprüche 4 bis 7 in das Genom eines nichtmenschlichen Säugetiers chromosomal eingebaut wird.

## Revendications

1. Polypeptide contenant une séquence d'acides aminés contigus de la séquence polypeptidique codée par l'exon 17 de l'ADNc du gène de l'APP 770, ladite séquence d'acides aminés contigus étant telle que :
- elle a de 5 au nombre total d'acides aminés codés par ledit exon 17,
- et elle contient l'acide aminé correspondant au codon 692 dans l'ADNc du gène de l'APP 770 et qui est l'alanine substituée par la glycine.

2. Polypeptide contenant une séquence d'acides aminés contigus de la β-amyloïde, ladite séquence d'acides aminés contigus étant telle que :
- elle contient de 5 au nombre total d'acides aminés de la β-amyloïde,
- et elle contient l'acide aminé correspondant au codon 692 dans l'ADNc du gène de l'APP 770 et qui est l'alanine substituée par la glycine.

3. Polypeptide contenant une séquence d'acides aminés contigus du transcrit APP 770, ladite séquence d'acides aminés contigus étant telle que :
- elle contient de 5 au nombre total d'acides aminés du transcrit APP 770, et
- elle contient l'acide aminé correspondant au codon 692 dans l'ADNc du gène de l'APP 770 et qui est l'alanine substituée par la glycine,
et plus particulièrement le polypeptide suivant :
Val-Phe-Phe-Gly-Glu-Asp-Val-Gly,
ou un polypeptide de 30 acides aminés contenant le susdit polypeptide.

4. Séquence d'acide nucléique caractérisée par le fait qu'elle code pour l'un quelconque des polypeptides selon les revendications 1 à 3.

5. Acide nucléique contenant une séquence nucléotidique de nucléotides contigus de l'exon 17, ladite séquence nucléotidique de nucléotides contigus étant telle que :
- elle a de 10 au nombre total de nucléotides de l'exon 17 de l'ADNc du gène de l'APP 770,
- et elle contient le nucléotide correspondant à la position 2075 dans l'ADNc du gène de l'APP 770, et qui est C substituée par G.

6. Acide nucléique contenant une séquence nucléotidique de nucléotides contigus des exons 16 et 17, ladite séquence nucléotidique de nucléotides contigus étant telle que :
- elle a de 10 au nombre total de nucléotides des exons 16 et 17 de l'ADNc du gène de l'APP 770,
- et elle contient le nucléotide correspondant à la position 2075 dans l'ADNc du gène de l'APP 770, et qui est C substituée par G.

7. Acide nucléique contenant une séquence nucléotidique de nucléotides contigus de l'ADNc du gène de l'APP 770, ladite séquence nucléotidique de nucléotides contigus étant telle que :
- elle a de 10 au nombre total de nucléotides de l'ADNc du gène de l'APP 770,
- et elle contient le nucléotide correspondant à la position 2075 dans l'ADNc du gène de l'APP 770, et qui est C substituée par G,
et plus particulièrement les oligonucléotides suivants :
GTTCTTTGGAGAAGATG
TTCTTTGGAGAAGAT,
ou des acides nucléiques contenant les susdits oligonucléotides.

8. Acide nucléique contenant une séquence nucléotidique selon l'une quelconque des revendications 4 à 7 insérée dans un acide nucléique hétérologue.

9. Vecteur recombinant, en particulier de clonage et/ou d'expression, comprenant une séquence vecteur, notablement du type plasmide, comside, ADN phagique ou ADN viral et un acide nucléique recombinant selon l'une quelconque des revendications 4 à 7, dans l'un des sites non essentiels pour sa réplication.

10. Vecteur recombinant selon la revendication 9, contenant dans l'un de ses sites non essentiels pour sa réplication des éléments nécessaires pour favoriser l'expression des polypeptides selon l'une quelconque des revendications 1 à 3 dans un hôte cellulaire et en particulier un promoteur reconnu par l'ARN polymérase de l'hôte cellulaire, en particulier un promoteur inductible et éventuellement une séquence codant pour les signaux de terminaison de la transcription et éventuellement une séquence signal et/ou une séquence d'ancrage.

11. Vecteur recombinant selon la revendication 10, contenant les éléments permettant l'expression par E. coli d'un acide nucléique selon l'une des revendications 4 à 7 insérés dans le vecteur, et en particulier les éléments permettant l'expression de l'acide nucléique selon l'une quelconque des revendications 1 à 4, ou une partie de celui-ci en tant que protéine mature ou en tant que partie d'une protéine de fusion contenant les signaux nécessaires pour le transport vers l'espace périplasmique, lesdits éléments contenant éventuellement des séquences d'acides aminés pour faciliter la maturation en aval de la protéine codée par ledit vecteur recombinant.

12. Hôte cellulaire qui est transformé par un vecteur recombinant selon l'une quelconque des revendications 10 ou 11, et contenant les éléments de régulation permettant l'expression d'une séquence nucléotidique codant pour le polypeptide selon l'une quelconque des revendications 1 à 3 dans cet hôte, comme une protéine mature ou comme une partie d'une protéine de fusion, la moitié de fusion qui est utilisée dans la protéine de fusion étant une partie d'une protéine non homologue choisie pour optimiser l'expression de la protéine de fusion.

13. Produit d'expression d'un acide nucléique selon l'une quelconque des revendications 4 à 7, exprimé par un hôte cellulaire transformé selon l'une quelconque des revendications 12 ou 13.

14. Sondes nucléotidiques, s'hybridant avec un acide nucléique selon l'une quelconque des revendications 4 à 7 ou avec leurs séquences complémentaires, dans des conditions d'hybridation telles qu'elles ne s'hybrident pas avec le gène d'APP non muté (en position 2075 du nucléotide) ou avec l'exon 17 non muté (en position 2075 du nucléotide) ou avec un ARN messager du gène de l'APP 770 non muté (en position 2075 du nucléotide) ou avec l'ARN messager de l'exon 17 non muté (en position 2075 du nucléotide), et plus particulier les suivants :
GTTCTTTGGAGAAGATG
TTCTTTGGAGAAGAT.

15. Méthode de détermination de la présence ou de l'absence de la mutation en position 2075 du nucléotide (par rapport à l'ADNc du gène de l'APP 770) dans le gène de l'APP 770 ou une partie de celui-ci, comprenant les étapes suivantes :
- le prélèvement d'un échantillon biologique d'un patient susceptible de contenir les séquences nucléotidiques selon l'une quelconque des revendications 4 à 7,
- l'amplification éventuelle des séquences nucléotidiques selon l'une quelconque des revendications 4 à 7 à l'aide d'un jeu d'amorces d'ADN,
- la mise en contact du susdit échantillon biologique ou des séquences nucléotidiques amplifiées contenues dans ce dernier avec une sonde nucléotidique selon la revendication 16, dans des conditions permettant la production d'un complexe d'hybridation formé entre ladite sonde et ladite séquence nucléotidique,
- la détection du susdit complexe d'hybridation éventuellement formé,
- faisant la conclusion quant à la présence ou l'absence de la susdite mutation.

16. Méthode de détermination de la présence ou de l'absence de la mutation en position 2075 du nucléotide (par rapport à l'ADNc du gène de l'APP 770) dans le gène de l'APP 770 ou une partie de celui-ci, comprenant les étapes suivantes :
- le prélèvement d'un échantillon biologique d'un patient susceptible de contenir les séquences nucléotidiques selon l'une quelconque des revendications 4 à 7,
- l'amplification d'une séquence nucléotidique selon l'une quelconque des revendications 4 à 7 à l'aide d'un jeu d'amorces d'ADN en présence de nucléotides radioactifs,
- la dénaturation de la séquence nucléotidique amplifiée pour la séparer en deux brins,
- le séquençage d'un brin de la séquence nucléotidique dénaturée obtenue,
- et éventuellement la détermination de la séquence dudit brin de la séquence nucléotidique contenant la mutation par séquençage du brin opposé,
- faisant la conclusion quant à la présence ou l'absence de la susdite mutation.

17. Méthode de détermination de la présence ou de l'absence de la mutation en position 2075 du nucléotide (par rapport à l'ADNc du gène de l'APP 770) dans le gène de l'APP 770 ou une partie de celui-ci, comprenant les étapes suivantes :
- le prélèvement d'un échantillon biologique d'un patient susceptible de contenir les séquences nucléotidiques selon l'une quelconque des revendications 4 à 7,
- l'amplification d'une séquence nucléotidique selon l'une quelconque des revendications 4 à 7 à l'aide d'un jeu d'amorces d'ADN,
- la dénaturation de la séquence nucléotidique amplifiée pour la séparer en deux brins,
- et la séparation de bandes, l'une correspondant au brin de la séquence nucléotidique contenant la mutation et l'autre correspondant au brin de la séquence nucléotidique ne contenant pas de mutation,
- faisant la conclusion quant à la présence ou l'absence de la susdite mutation.

18. Méthode de détermination de la présence ou de l'absence de la mutation en position 2075 du nucléotide (par rapport à l'ADNc du gène de l'APP 770) dans le gène de l'APP 770 ou une partie de celui-ci, comprenant les étapes suivantes :
- le prélèvement d'un échantillon biologique d'un patient susceptible de contenir les séquences nucléotidiques selon l'une quelconque des revendications 4 à 7,
- l'amplification des séquences nucléotidiques selon l'une quelconque des revendications 4 à 7 à l'aide d'un jeu d'amorces d'ADN,
- la digestion avec l'enzyme de restriction CviRI susceptible de cliver le site CviRI, qui est présent dans la forme non mutée et absent dans la forme mutée,
- la séparation des bandes provenant de la digestion, par exemple sur un gel de polyacrylamide ou un gel d'agarose et
- la détection des bandes, par exemple par le bromure d'éthidium,
- faisant la conclusion quant à la présence ou l'absence de la susdite mutation.

19. Méthode de détermination de la présence ou de l'absence de la mutation en position 2075 du nucléotide (par rapport à l'ADNc du gène de l'APP 770) dans le gène de l'APP 770 ou une partie de celui-ci, comprenant les étapes suivantes :
- le prélèvement d'un échantillon biologique d'un patient susceptible de contenir des polypeptides selon l'une quelconque des revendications 1 à 3,
- le séquençage de l'acide aminé de l'une quelconque des revendications 1 à 3,
- faisant la conclusion quant à la présence ou l'absence de la susdite mutation.

20. Méthode de production d'un animal mammifère non humain transgénique ayant une probabilité de développer un dépôt de plaques amyloïdes et/ou une hémorragie cérébrale, ladite méthode comprenant l'incorporation chromosomique d'une séquence nucléotidique selon l'une quelconque des revendications 4 à 7 dans le génome d'un animal mammifère non humain.
